# EUROPEAN PATENT SPECIFICATION

(11) **EP 2 225 052 B1**
(45) Date of publication and mention of the grant of the patent: **01.05.2013**
(21) Application number: 08852961.5
(22) Date of filing: 20.11.2008
(51) Int. Cl.: B08B 15/00, B09B 1/00, F17D 1/04, C12M 1/00, C12M 1/107

(54) **SYSTEM FOR COLLECTING BIOGAS GENERATED BY WASTE**
SYSTEM ZUM SAMMELN VON DURCH ABFALL ERZEUGTEM BIOGAS
SYSTÈME POUR LA COLLECTE DE BIOGAZ GÉNÉRÉ PAR DES DÉCHETS

(30) Priority: 21.11.2007 IT BO20070769
(43) Date of publication of application: 08.09.2010
(73) Proprietor: Sandrini, Francesca, Cesana (IT)
(72) Inventor: Sandrini, Francesca, Cesana (IT)
(74) Representative: Manconi, Stefano
(86) International application number: PCT/IB2008/003152
(87) International publication number: WO 2009/066157

(56) References cited:
- DE-A1- 10 352 093
- US-A- 5 287 927
- US-B1- 6 254 524

## Description

### TECHNICAL FIELD

The present invention relates to a system for collecting biogas generated by waste.

### BACKGROUND ART

In the field of waste disposal, it is known to extract the biogas generated by natural fermentation of masses of urban solid waste contained within respective storage wells and supply the biogas extracted from the storage wells to a collection system.

Generally, the collection system comprises, as shown in Document US-A-5287927, a plurality of collection units mounted in parallel to one another, each of which comprises an inlet coupling of the biogas extracted from a corresponding storage well and a duct for supply of the biogas to a collection manifold, which is connected in a fluid-dynamic way with the supply ducts, and is mounted, together with the collection units, on a metal supporting structure.

The known collection systems of the type described above present certain drawbacks principally deriving from the fact that they entail the construction of the aforesaid supporting structure, its transportation to the installation site, and the pre-arrangement of various supporting structures according to the number of collection units each time necessary, and have, moreover, a relatively reduced versatility and flexibility.

### DISCLOSURE OF INVENTION

It is an object of the present invention to provide a system for collecting biogas generated by waste that will be free from the drawbacks set forth above and that will be simple and inexpensive to provide.

According to the present invention, there is provided a system for collecting biogas generated by waste according to what is claimed in the annexed claims.

### BRIEF DESCRIPTION OF THE DRAWINGS

The present invention will now be described with reference to the annexed drawings, which illustrate a non-limiting example of embodiment thereof and in which:
Figure 1 is a schematic perspective view of a preferred embodiment of the collection system according to the present invention;
Figure 2 is a side view of a first detail of Figure 1;
Figure 3 is a perspective view of a second detail of Figure 1;
Figure 4 is a side view of a third detail of Figure 1; and
Figure 5 is a side view of a fourth detail of Figure 1.

### BEST MODE FOR CURRYING OUT THE INVENTION

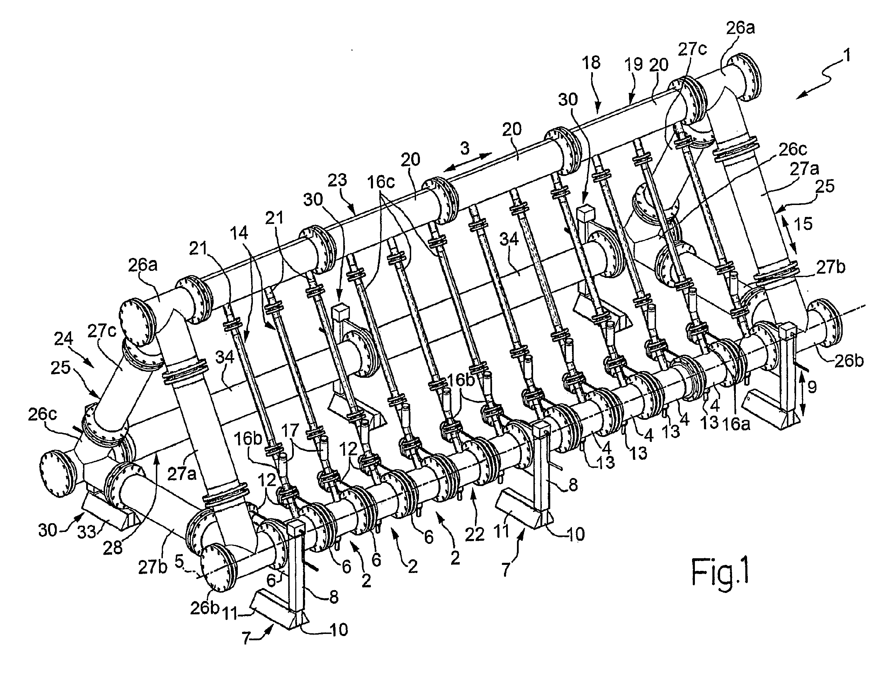

With reference to Figure 1, designated as a whole by 1 is a system for collecting biogas generated by natural fermentation of masses of urban solid waste (not illustrated) stored in respective storage wells (known and not illustrated).

The system 1 comprises a plurality of collection units 2 (in the case in point twelve collection units 2), which are equal in number to the aforesaid storage wells (not illustrated), are associated, each, to a corresponding storage well (not illustrated), are mounted in parallel with respect to one another, and are aligned to one another in a given direction 3.

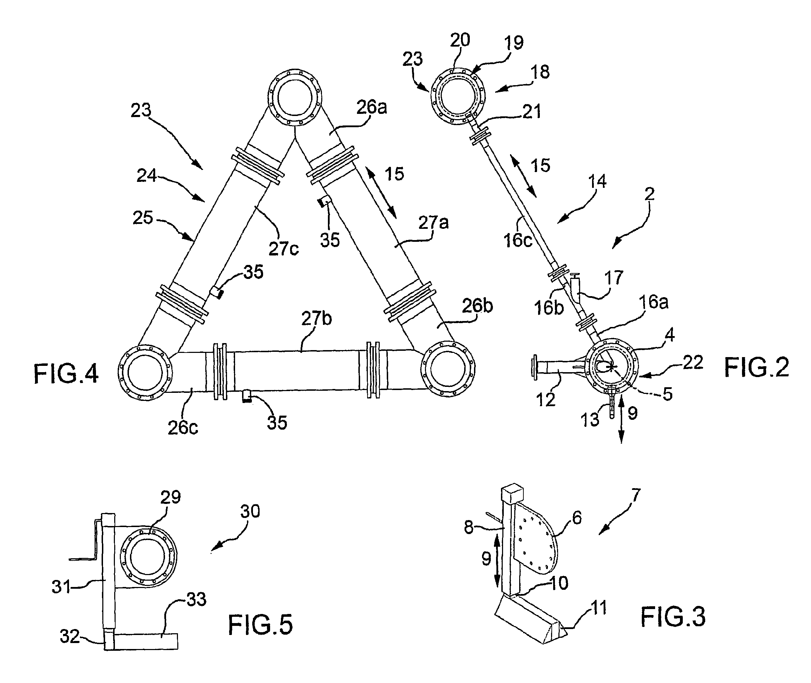

According to what is illustrated in Figures 1, 2, and 3, each unit 2 comprises a substantially cylindrical inlet coupling 4, which has a longitudinal axis 5 parallel to the direction 3, is aligned to the couplings 4 of the other units 2 in the direction 3, is mechanically connected to each adjacent coupling 4, and is fluid-dynamically separated from each adjacent coupling 4 via a substantially circular plate 6 mounted between the two couplings 4 in a direction orthogonal to the axis 5.

The plate 6 set between the two central couplings 4 defines part of a resting device 7 further comprising a box-type guide 8, which is fixed to the plate 6, extends in a direction 9 transverse to the direction 3, and is engaged in a slidable way by a slide 10 provided with a resting foot 11 mounted in a position corresponding to a bottom free end of the slide 10 projecting on the outside of the guide 8.

In connection with what has been set forth above it should be pointed out that also the free end of each of the two external couplings 4 is closed in the direction 3 by the plate 6 of a resting device 7 altogether equivalent to the resting device 7 described above.

The position of the slides 10 and hence of the feet 11 along the corresponding guides 8 in the direction 9 is controlled selectively to maintain the directions 3 and 9 horizontal and vertical, respectively, and thus guarantee a proper positioning of the system 1 on the ground.

Each coupling 4 is provided with a radial inlet duct 12, which extends in a transverse direction with respect to the axis 5, and is connected to the corresponding storage well (not illustrated) to supply within the coupling 4 the biogas generated by the waste stored in the storage well (not illustrated) itself, and with a bottom discharge duct 13, which extends in the direction 9 and projects radially downwards from the outer surface of the coupling 4 for discharging the condensate generated by the biogas fed through the coupling 4 itself on the outside of the unit 2.

Each unit 2 further comprises a supply duct 14, which projects radially outwards from the corresponding coupling 4, extends in a direction 15 inclined by an angle other than 0°, preferably approximately 30°, with respect to the direction 9, is parallel to the ducts 14 of the other units 2, and comprises, in the case in point, three portions 16a, 16b, 16c connected to one another in a fluid-dynamic way, and of which the portion 16a is an inlet portion fluid-dynamically connected to the corresponding coupling 4, the portion 16b is an intermediate portion provided with a valve 17 of a known type for regulating the flowrate, and the portion 16c is an outlet portion.

With reference to Figure 1, the system 1 further comprises a manifold 18 for collecting the biogas fed through the couplings 4 and along the ducts 14.

In the case in point, the manifold 18 comprises a main duct 19, which extends in the direction 3, and in turn comprises four portions 20 connected to one another in a fluid-dynamic way. The manifold 18 further comprises for each duct 14, a secondary duct 21, which extends in the direction 15, and is connected in a fluid-dynamic way to the duct 19 and to the duct 14 itself.

The couplings 4, the resting devices 7, and the manifold 18 define a first portion 22 of a supporting structure 23 of the system 1 further comprising a second portion 24 designed to stabilize the first portion 22 and comprising, in turn, two tubular supporting frames 25, which are set on opposite sides of the couplings 4 and of the manifold 18 in the direction 3, and have a substantially triangular shape.

According to what is illustrated in Figures 1, 4, and 5, each frame 25 comprises three four-way pipe fittings 26 connected in a fluid-dynamic way to one another in twos via respective intermediate ducts 27, of which one (designated in what follows by 27a) is parallel to the direction 15, one (designated in what follows by 27b) is substantially orthogonal to the directions 3 and 9, and one (designated in what follows by 27c) is set according to an angle of substantially 60° with respect to the ducts 27a and 27b.

One of the pipe fittings 26 (in what follows designated by 26a) is connected in a fluid-dynamic way to a corresponding free end of the manifold 18, one (in what follows designated by 26b) is connected to a corresponding external coupling 4 via the interposition of the plate 6 of the corresponding resting device 7, and one (designated in what follows by 26c) is connected in a fluid-dynamic way to a corresponding free end of a duct 28, which extends between the two pipe fittings 26c parallel to the direction 3, and is substantially coplanar to the couplings 4.

Each pipe fitting 26c is connected to the duct 28 via the interposition of an annular disk 29 defining part of a corresponding resting device 30 further comprising a box-type guide 31, which is fixed to the disk 29, extends in the direction 9, and is engaged in a slidable way by a slide 32 provided with a resting foot 33 mounted in a position corresponding to a bottom free end of the slide 32 projecting on the outside of the guide 31.

The duct 28 comprises, in the case in point, two portions 34 connected to one another via the interposition of the disk 29 of a resting device 30 altogether equivalent to the resting device 30 described above.

The position of the slides 32 and hence of the feet 33 along the corresponding guides 31 in the direction 9 is controlled selectively, in a way similar to what has been described for the resting devices 7, to maintain the directions 3 and 9 horizontal and vertical, respectively, and hence guarantee a correct positioning of the system 1 on the ground.

Each frame 25 is moreover provided, in the case in point, with three discharge ducts 35 set at different heights in the direction 9 for discharging selectively on the outside of the system 1 part, or all, of the condensate generated by the biogas fed through the frames 25 and the duct 28.

In use, the biogas generated within the aforesaid storage wells (not illustrated) is fed in succession through the couplings 4, along the ducts 14, along the manifold 18, through the frames 25, and along the duct 28, and is finally discharged on the outside of the system 1 via at least one outlet (not illustrated) made in the manifold 18 and/or in the duct 28 and/or in one of the frames 25. The condensate generated by the biogas within the couplings 4 is discharged on the outside of the system 1 via the corresponding ducts 13, whilst the condensate generated by the biogas within the frames 25 and the duct 28 is discharged on the outside of the system 1 via the ducts 35.

From what has been set forth above it follows that the fluid-dynamic connection between the manifold 18 and the frames 25 and between the frames 25 and the duct 28 enables the couplings 4 and the manifold 18 to define part of the supporting structure 23 and the biogas and/or the condensate generated by the biogas itself to flow within the frames 25 and the duct 28 and to bestow on the supporting structure 23 a weight sufficient for stabilizing the entire system 1, preventing the use of an auxiliary and independent supporting structure on which the units 2 and the manifold 18 are mounted.

The structure 23 is, then, a self-supporting modular structure, which integrates inside it the couplings 4 and the manifold 18, has a relatively high versatility and flexibility, is symmetrical with respect to a substantially vertical plane of symmetry containing the manifold 18, and can be mounted directly on the installation site of the system 1, thus reducing considerably the costs of storage and transportation of the structure 23 itself.

According to variants (not illustrated):
each duct 14 and/or the manifold 18 and/or each frame 25 and/or the duct 28 are made in a single piece;
the frames 25 are separate from the manifold 18, and the second portion 24 of the structure 23 is filled and stabilized via a further fluid fed within the second portion 24;
the couplings 4 are fluid-dynamically connected to one another; and
the external couplings 4 are fluid-dynamically connected to the frames 25.

## Claims

1. A system for collecting biogas generated by waste, the system comprising a supporting structure (23); a plurality of collection units (2), each of which comprises, in turn, a tubular inlet coupling (4) of the biogas and a supply duct (14) of the biogas itself; and a tubular manifold (18) for collection of the biogas fed along the supply ducts (14), each supply duct (14) extending from the corresponding inlet coupling (4) to the manifold (18); and being **characterized in that**:
the inlet couplings (4) are aligned to one another in a first direction (3), and are mechanically connected to one another in series;
the collection manifold (18) is parallel to the series of inlet couplings (4), and defines, together with the inlet couplings (4), a first portion (22) of the supporting structure (23); and
the supporting structure (23) further comprises a second portion (24), which is at least in part tubular and communicates in a fluid-dynamic way with the collection manifold (18) allowing the biogas to flow in the second portion (24) and to stabilize the first portion (22) and the second portion (24).

2. The system according to claim 1, wherein each collection unit (2) further comprises a regulating valve (17) for regulating the flowrate of the biogas fed through the collection unit (2) itself.

3. The system according to any one of the preceding claims, wherein each collection unit (2) further comprises at least one discharge (13) for discharge of the condensate generated by the biogas fed through the collection unit (2) itself.

4. The system according to any one of the preceding claims, wherein each supply duct (14) is inclined according to an angle other than 0° with respect to a substantially vertical second direction (9).

5. The system according to any one of the preceding claims, wherein the second portion (24) comprises two tubular supporting frames (25) of a substantially triangular shape set on opposite sides of the collection manifold (18) and of the collection units (2) in the first direction (3).

6. The system according to Claim 5, wherein each supporting frame (25) is connected to a corresponding inlet coupling (4), and is connected, moreover, in a fluid-dynamic way, to a corresponding free end of the collection manifold (18).

7. The system according to Claim 5 or 6, wherein each supporting frame (25) comprises a plurality of first ducts (26, 27) connected to one another.

8. The system according to any one of Claims 5 to 7, wherein each supporting frame (25) is provided with at least one further drain (35) for discharging the condensate generated by the biogas fed through the supporting frame (25) itself.

9. The system according to Claim 8, wherein each supporting frame (25) is provided with a plurality of said further discharge outlets (35), set at different heights with respect to one another.

10. The system according to any one of Claims 5 to 9, wherein the second portion (24) further comprises a further supply duct (28), which extends parallel to the collection manifold (18) and to the inlet couplings (4) and is connected in a fluid-dynamic way to the supporting frames (25).

11. The system according to any one of the preceding claims, wherein the supporting structure (23) further comprises a plurality of adjustable supporting devices (7, 30), set underneath the first portion and/or the second portion (22, 24) to guarantee proper resting of the system on the ground.

12. The system according to any one of the preceding claims, wherein the inlet couplings (4) are mechanically connected to one another and are fluid-dynamically separated from one another via respective separator diaphragms (6).

## Patentansprüche

1. Ein System zum Sammeln von Biogas, das aus Abfall erzeugt wurde, wobei das System umfasst: eine Unterstützungsstruktur (23), eine Mehrzahl von Sammeleinheiten (2), von dehnen jede wiederum eine rohrförmige Einlasskupplung (4) des Biogases und eine Versorgungsleitung (14) des Biogases selbst aufweist, sowie einen rohrförmigen Verteiler (18) zum sammeln der Biogaszuführung entlang der Versorgungsleitungen (14), wobei sich jede Versorgungsleitung (14) von der entsprechenden Einlasskupplung (4) zu dem Verteiler (18) erstreckt, **dadurch gekennzeichnet, dass**
die Einlasskupplungen (4) miteinander in einer ersten Richtung (3) ausgerichtet sind und mechanisch miteinander in Reihe verbunden sind,
der Sammelverteiler (18) ist parallel zu der Reihe von Einlasskupplungen (4) angeordnet und definiert, zusammen mit den Einlasskupplungen (4), einen ersten Bereich (22) der Unterstützungsstruktur (23), und
die Unterstützungsstruktur (23) umfasst weiterhin einen zweiten Bereich (24), der mindestens zum Teil rohrförmig ist und auf eine fluid-dynamische Weise mit dem Sammelverteiler (18) in Verbindung steht, was es erlaubt, dass das Biogas in den zweiten Bereich (24) strömt und dass der erste Bereich (22) und der zweite Bereich (24) stabilisiert werden.

2. Das System gemäß Patentanspruch 1, wobei jede Sammeleinheit (2) weiterhin ein Regelventil (17) zum Regeln der Durchflussmenge der Biogaszuführung durch die Sammeleinheit (2) selbst aufweist.

3. Das System gemäß einem der vorhergehenden Patentansprüche, wobei jede Sammeleinheit (2) weiterhin mindestens einen Auslass (13) zum Auslassen des Kondensats umfasst, das durch die Biogaszuführung durch die Sammeleinheit (2) selbst erzeugt wird.

4. Das System gemäß einem der vorhergehenden Patentansprüche, wobei jede Versorgungsleitung (14) in einen Winkel geneigt ist, der von 0 ° in Bezug auf eine im Wesentlichen vertikale zweite Richtung (9) verschieben ist.

5. Das System gemäß einem der vorhergehenden Patentansprüche, wobei der zweite Bereich (24) zwei rohrförmige Unterstützungsrahmen (25) eines im Wesentlichen dreieckigen Formsatzes auf entgegengesetzten Seiten des Sammelverteilers (18) und der Sammeleinheiten (2) in der ersten Richtung (3) umfasst.

6. Das System gemäß Patentanspruch 5, wobei jeder Unterstützungsrahmen (25) mit einer entsprechenden Einlasskupplung (4) verbunden ist, und darüber hinaus in einer fluid-dynamischen Weise mit einem entsprechenden freien Ende des Sammelverteilers (18) verbunden ist.

7. Das System gemäß Patentanspruch 5 oder 6, wobei jeder Unterstützungsrahmen (25) eine Mehrzahl von ersten Leitungen (26, 27) umfasst, die miteinander verbunden sind.

8. Das System gemäß einem der Patentansprüche 5 bis 7, wobei jeder Unterstützungsrahmen (25) mit mindestens einem weiteren Ablauf (35) zum Auslassen des Kondensats bereitgestellt ist, das durch die Biogaszuführung durch den Unterstützungsrahmen (25) selbst erzeugt wird.

9. Das System gemäß Patentanspruch 8, wobei jeder Unterstützungsrahmen (25) bereitgestellt ist mit einer Mehrzahl der weiteren Auslassabläufe (35), die in verschiedenen Höhen in Bezug aufeinander angeordnet sind.

10. Das system gemäß einem der Patentansprüche 5 bis 9, wobei der zweite Bereich (24) weiterhin eine weitere Versorgungsleitung (28) umfasst, die sich parallel zum Sammelverteiler (18) und zu den Einsatzkupplungen (4) erstreckt und in einer fluid-dynamischen Weise mit dem Unterstützungsrahmen (25) verbunden ist.

11. Das System gemäß einem der vorhergehenden Patentansprüche, wobei die Unterstützungsstruktur (23) weiterhin eine Mehrzahl von einstellbaren Unterstützungsvorrichtungen (7, 30) umfasst, die unterhalb des ersten Bereichs und/oder des zweiten Bereichs (22, 24) angeordnet sind, um ein ordnungsgemäßes Aufliegen des Systems auf dem Boden zu garantieren.

12. Das System gemäß einem der vorhergehenden Patentansprüche, wobei die Einlasskupplungen (4) mechanisch miteinander verbunden sind und fluid-dynamisch über entsprechende Trennmembranen (6) voneinander getrennt sind.

## Revendications

1. Système pour la collecte de biogaz générés par des déchets, le système comprenant une structure de support (23) ; une pluralité d'unités de collecte (2) comprenant chacune, à son tour, un accouplement d'entrée tubulaire (4) du biogaz et un conduit d'alimentation (14) du biogaz ; et un collecteur tubulaire (18) pour la collecte du biogaz fourni par les conduits d'alimentation (14), chaque conduit d'alimentation (14) s'étendant de l'accouplement d'entrée (4) correspondant au collecteur (18) ; et **caractérisé en ce que**
les accouplements d'entrée (4) sont alignés entre eux dans une première direction (3) et sont mécaniquement reliés l'un à l'autre en série ;
le collecteur de collecte (18) est parallèle à la série d'accouplements d'entrée (4) et il définit, avec les accouplements d'entrée (4), une première portion (22) de la structure de support (23) ; et
la structure de support (23) comprend en outre une deuxième portion (24) qui est au moins partiellement tubulaire et qui est en communication fluidique avec le collecteur de collecte (18) pour permettre au biogaz de s'écouler dans la deuxième portion (24) et de stabiliser la première portion (22) et la deuxième portion (24).

2. Système selon la revendication 1, dans lequel chaque unité de collecte (2) comprend en outre une vanne de régulation (17) pour réguler le débit du biogaz fourni par l'unité de collecte (2).

3. Système selon l'une quelconque des revendications précédentes, dans lequel chaque unité de collecte (2) comprend en outre au moins une évacuation (13) pour évacuer la condensation générée par le biogaz fourni par l'unité de collecte (2).

4. Système selon l'une quelconque des revendications précédentes, dans lequel chaque conduit d'alimentation (14) est incliné à un angle différent de 0° par rapport à une deuxième direction sensiblement verticale (9).

5. Système selon l'une quelconque des revendications précédentes, dans lequel la deuxième portion (24) comprend deux cadres de support tubulaires (25) de forme sensiblement triangulaire placés sur des côtés opposés du collecteur de collecte (18) et des unités de collecte (2) dans la première direction (3).

6. Système selon la revendication 5, dans lequel chaque cadre de support (25) est relié à un accouplement d'entrée correspondant (4), et est relié en outre, en communication fluidique, à une extrémité libre correspondante du collecteur de collecte (18).

7. Système selon la revendication 5 ou 6, dans lequel chaque cadre de support (25) comprend une pluralité de premiers conduits (26, 27) reliés l'un à l'autre.

8. Système selon l'une quelconque des revendications 5 à 7, dans lequel chaque cadre de support (25) est pourvu d'au moins un autre drain (35) pour évacuer la condensation générée par le biogaz fourni par l'intermédiaire du cadre de support (25).

9. Système selon la revendication 8, dans lequel chaque cadre de support (25) est pourvu d'une pluralité desdites autres sorties d'évacuation (35) placées à des hauteurs différentes l'une de l'autre.

10. Système selon l'une quelconque des revendications 5 à 9, dans lequel la deuxième portion (24) comprend en outre un autre conduit d'alimentation (28) qui s'étend parallèlement au collecteur de collecte (18) et aux accouplements d'entrée (4) et est relié, en communication fluidique, aux cadres de support (25).

11. Système selon l'une quelconque des revendications précédentes, dans lequel la structure de support (23) comprend en outre une pluralité de dispositifs de support ajustables (7, 30) placés au-dessous de la première portion et/ou de la deuxième portion (22, 24) pour garantir une assise appropriée du système au sol.

12. Système selon l'une quelconque des revendications précédentes, dans lequel les accouplements d'entrée (4) sont reliés mécaniquement l'un à l'autre et sont séparés fluidiquement l'un de l'autre par l'intermédiaire de diaphragmes séparateurs respectifs (6).
